# EUROPÄISCHE PATENTANMELDUNG

(11) **EP 3 000 392 A1**
(43) Veröffentlichungstag der Anmeldung: **30.03.2016**
(21) Anmeldenummer: 15181704.6
(22) Anmeldetag: 20.08.2015
(51) Int. Cl.: A61B 5/08, A61B 5/097, A61B 5/087, A61M 16/00, A62B 9/00, A62B 9/02, A62B 9/06, A62B 18/00, A62B 18/08, A62B 18/10, A62B 27/00

(54) **VORRICHTUNG ZUR ANALYSE DER AUSATEMLUFT UND VERWENDUNG DER VORRICHTUNG**

(30) Priorität: 23.09.2014 DE 102014219161
(71) Anmelder: ROBERT BOSCH GMBH, 70442 Stuttgart (DE)
(72) Erfinder: Reisinger, Daniel, 72108 Rottenburg-Wendelsheim (DE); Zeyher, Peter, 64289 Darmstadt (DE); Barth, Frank, 71732 Tamm (DE); Wingsch, Volker, 72762 Reutlingen (DE); Giezendanner-Thoben, Robert, 70839 Gerlingen (DE)

(57) **Zusammenfassung**

Bei einer Vorrichtung (200) zur Analyse der Ausatemluft umfasst die Vorrichtung eine Gassensoreinrichtung (110). Die Vorrichtung ist insbesondere zur Messung von Stickstoffoxiden in der Ausatemluft vorgesehen. Zur Führung von Luft in der Vorrichtung ist ein Hauptgaspfad (130) vorgesehen, von dem wenigstens ein Messgaspfad (240) und wenigstens ein Spülgaspfad (250) abzweigen.

## Beschreibung

Die vorliegende Erfindung betrifft eine Vorrichtung zur Analyse der Ausatemluft und insbesondere zu Messung von Stickstoffoxiden in der Ausatemluft, wobei die Vorrichtung eine Gassensoreinrichtung mit wenigstens einem Gassensor umfasst. Darüber hinaus betrifft die Erfindung eine Verwendung der Vorrichtung.

### Stand der Technik

Es werden bereits verschiedene Geräte für eine Atemgasanalyse für medizinische Zwecke, insbesondere zur Diagnostik, und auch für verschiedene Lifestyle-Anwendungen eingesetzt. Im Hinblick auf asthmatische Erkrankungen spielt endogenes Stickstoffmonoxid in der Ausatemluft einer Person eine wichtige Rolle. Stickstoffmonoxid ist ein Indikator für entzündliche Reaktionen in den Atemwegen und insbesondere in der Lunge. Es ist bereits bekannt, den Stickstoffmonoxidgehalt in der Atemluft zu überwachen, um ein verbessertes Management bei Asthmapatienten zu erreichen. Zu diesem Zweck sind verschiedene Atemluftanalysegeräte bekannt. Beispielsweise beschreibt die US-Patentanmeldung US 2010/0192669 A1 ein Atemluftanalysegerät mit einem photoakustischen Probendetektor, mit dem Stickstoffmonoxid in der Ausatemluft bestimmt werden kann. Die deutsche Gebrauchsmusterschrift DE 20 2009 018 824 U1 offenbart ein Probeentnahmesystem für ein Gerät zur Atemgasanalyse, bei dem der Fluss der Atemluft des Patienten durch das Messgerät so gesteuert wird, dass der klinisch entscheidende Anteil des Atemvolumens über einen Messsensor geleitet werden kann. Hierbei weist das Analysegerät ein Leitungssystem für das Atemgas mit einer Verzweigung auf, wobei ein erster Leitungszweig zu einer Messkammer mit einem Gassensor und ein zweiter Leitungszweig zu einer weiteren Auslassöffnung führen. Zur Spülung der Messkammer kann Umgebungsluft über einen Filter und ein Gebläse in die Messkammer geleitet werden. Die Veröffentlichungsschrift WO 02/088691 A2 einer internationalen Patentanmeldung beschreibt eine Vorrichtung zur Messung von Stickstoffmonoxid in der Ausatemluft, wobei ein Gassensor eingesetzt wird, der nach dem Prinzip der Austrittsarbeitsmessung betrieben wird. Hierbei ist ein Oxidationskatalysator als Konverter vorgesehen, der Stickstoffmonoxid zu Stickstoffdioxid oxidiert. Aus der gemessenen Stickstoffdioxidkonzentration kann auf die Stickstoffmonoxidkonzentration in der Ausatemluft geschlossen werden. Als Gassensoren werden hierbei Feldeffekttransistoren verwendet.

### Offenbarung der Erfindung

### Vorteile der Erfindung

Die Erfindung stellt eine Vorrichtung zur Analyse der Ausatemluft und insbesondere zur Messung von Stickstoffoxiden in der Ausatemluft, also ein Atemgasanalysegerät, bereit, mit dem in besonders vorteilhafter Weise der Stickstoffmonoxidgehalt in der Ausatemluft einer Person oder gegebenenfalls eines Tieres bestimmt werden kann. Die Vorrichtung umfasst eine Gassensoreinrichtung, insbesondere in Form einer Messkammer, die vorzugsweise wenigstens einen Gassensor zur Messung von Stickstoffoxiden umfasst. Erfindungsgemäß ist zur Führung von Luft, insbesondere der Ausatemluft, in der Vorrichtung ein Hauptgaspfad vorgesehen, von dem wenigstens ein Messgaspfad und wenigstens ein Spülgaspfad abzweigen. Sowohl der Messgaspfad als auch der Spülgaspfad münden in die Messkammer bzw. in die Gassensoreinrichtung. Zweckmäßigerweise wird nur ein Teil, beispielsweise 10 %, der im Hauptgaspfad geführten Ausatemluft in den Messgaspfad bzw. in den Spülgaspfad eingespeist. Der übrige Teil des Luftstromes verlässt die Vorrichtung über eine Auslassöffnung des Hauptgaspfades. Es wird also ein Teilstrom der in der Vorrichtung geführten Ausatemluft in die Messkammer bzw. in die Gassensoreinrichtung geleitet. Der Teilstrom, der über den Messgaspfad in die Messkammer geleitet wird, wird für die Messung der Stickstoffoxide verwendet. Der Teilstrom, der über den Spülgaspfad in die Messkammer geleitet wird, dient zum Spülen und/oder gegebenenfalls zum Kalibrieren und/oder zum Nulllinienabgleich der Gassensoreinrichtung bzw. des Gassensors.

In einer besonders bevorzugten Ausgestaltung der Vorrichtung ist in dem Spülgaspfad wenigstens ein Filter vorgesehen, mit dem in dem Spülgaspfad im Wesentlichen schadstofffreie Luft generiert wird. Bei dem Filter (Null-Luft-Filter) kann es sich beispielsweise um einen Aktivkohlefilter handeln. Die schadstofffreie Luft zeichnet sich insbesondere dadurch aus, dass im Wesentlichen keine Stickstoffoxide, insbesondere kein Stickstoffmonoxid oder Stickstoffdioxid enthalten sind. Weiterhin ist die schadstofffreie Luft vorzugsweise von alkoholischen Komponenten und/oder von Kohlenstoffmonoxid befreit. Durch die Filterung der Luft wird also eine im Wesentlichen schadstofffreie Luftfraktion generiert, die als Spülluft geeignet ist. Alternativ zu einer Verwendung von Ausatemluft für die Generierung der Spülluft kann auch Umgebungsluft mit Hilfe einer Pumpe insbesondere durch das Mundstück der Vorrichtung angesaugt werden. Durch das Spülen mit der im Wesentlichen schadstofffreien Luft kann die in der Messkammer verbliebene Atemluft nach dem Messvorgang ausgespült werden. Darüber hinaus kann der Gassensor kalibriert und/oder die Nulllinie des Gassensors neu eingestellt werden. In besonders bevorzugter Weise ist zur Entnahme von Gas, also insbesondere von Atemluft, aus dem Hauptgaspfad in den Messgaspfad und in den Spülgaspfad wenigstens eine Pumpe vorgesehen. Prinzipiell sind hierbei zwei Ausgestaltungen besonders vorteilhaft. In einer ersten Ausgestaltung kann eine gemeinsame Pumpe für den Messgaspfad und den Spülgaspfad vorgesehen sein. In einer zweiten Ausgestaltung ist sowohl im Messgaspfad als auch im Spülgaspfad jeweils wenigstens eine Pumpe vorgesehen. Mittels der oder den Pumpe(n) kann eine genaue Steuerung und Kontrolle der Probenentnahme im Hinblick auf den Zeitpunkt der Messung und das Probenvolumen erfolgen, wodurch die Messung vereinfacht und präzisiert werden kann. Das aktive Pumpen des Luftstroms bringt weiterhin den Vorteil mit sich, dass durch die hiermit verbundene Druckerhöhung im System der Querschnitt der verwendeten Leitungen und Ventile im Vergleich mit passiven Systemen kleiner ausgelegt werden kann.

In der Ausgestaltung mit einer Pumpe ist zweckmäßigerweise eine Umschaltung zwischen dem Messgaspfad und dem Spülgaspfad vorgesehen, wobei zu diesem Zweck vorzugsweise ein Umschaltventil in dem Leitungssystem angeordnet ist. Hierbei kann es sich beispielsweise um ein übliches elektromechanisches Umschaltventil handeln. Die gemeinsame Pumpe kann im Prinzip vor oder nach der Gassensoreinrichtung angeordnet sein, also stromaufwärts oder stromabwärts der Gassensoreinrichtung. Die Anordnung der Pumpe stromabwärts der Gassensoreinrichtung hat den besonderen Vorteil, dass eine Verschmutzung der Gassensoreinrichtung bzw. der Messkammer, die von der Pumpe verursacht sein kann, vermieden wird. Darüber hinaus kann bei einer stromabwärts der Gassensoreinrichtung angeordneten Pumpe ein Unterdruck in der Messkammer erzeugt werden. Hierdurch kann eine Spülung und die Regenerierung des Sensors in der Gassensoreinrichtung beschleunigt werden, sodass das System für die nächste Messung schneller wieder bereit ist.

In der anderen Ausgestaltung der Vorrichtung mit wenigstens einer Pumpe im Messgaspfad und wenigstens einer Pumpe im Spülgaspfad können der Gasfluss über den Messgaspfad und der Gasfluss über den Spülgaspfad in besonders effektiver Weise gesteuert und kontrolliert werden. In dieser Ausgestaltung kann vollständig auf ein Umschaltventil verzichtet werden. Durch die Verwendung von zwei günstigen Mikropumpen anstatt des in der anderen Variante erforderlichen Ventils können die Materialkosten um mehr als 30 % gesenkt werden. Auch der erforderliche Bauraum reduziert sich bei dieser Variante.

Bei der oder den Pumpe(n) im System kann es sich beispielsweise um Membranpumpen und/oder sogenannte *Microblower* handeln. *Microblower* sind miniaturisierte Luftpumpen, die mit piezokeramischen Elementen arbeiten. Diese Pumpen sind für kleine Volumenflüsse sehr geeignet und lassen sich darüber hinaus sehr genau steuern. Darüber hinaus kann die Bauweise sehr klein gehalten werden, sodass sich diese Pumpen für die Herstellung von handlichen Geräten besonders eignen.

Zweckmäßigerweise ist der Pumpe oder ist den Pumpen jeweils wenigstens ein Rückschlagventil zugeordnet. Je nach Art der Pumpe ist das Rückschlagventil stromaufwärts oder stromabwärts der Pumpe angeordnet. Bei einem *Microblower* als Pumpe kann das Rückschlagventil, das stromaufwärts oder stromabwärts des *Microblowers* angeordnet ist, vorteilhafterweise so ausgelegt werden, dass sich das Rückschlagventil gerade erst durch eine Druckerhöhung durch die laufende Pumpe öffnet, z. B. bei einem Öffnungsdruck von 0,3 kPa. Dadurch wird gewährleistet, dass nur bei laufender Pumpe ein Gasstrom in die Messkammer bzw. in die Sensoreinrichtung geleitet wird.

Für zuverlässige Messergebnisse und auch für die Benutzerfreundlichkeit ist eine gute Auslegung des Systems im Hinblick auf die Druckverhältnisse und die Strömungsmengen wichtig. Insbesondere soll für den Anwender oder den Patienten ein möglichst angenehmer Atemvorgang vollzogen werden können, wenn die Ausatemluft in das Gerät eingeblasen wird. Ein zu hoher Luftwiderstand oder eine abrupte Beendigung der Ausatmung durch einen Ventilverschluss kann für den Patienten im Gebrauch unangenehm sein und unter Umständen auch die Messergebnisse verfälschen. Um eine abrupte Beendigung der Ausatmung zu verhindern, sieht die erfindungsgemäße Vorrichtung vor, dass der Hauptgaspfad mit einer eigenen Auslassöffnung ausgestattet ist, sodass immer ein gewisser Durchfluss der Ausatemluft in dem Gerät sichergestellt ist. Für einen optimalen Betrieb der erfindungsgemäßen Vorrichtung ist beispielsweise ein Luftstrom im Hauptgaspfad von 50 ml/s bei einem Gegendruck für den Patienten von 0,5 bis 2 kPa besonders geeignet, wie es beispielsweise in der Richtlinie *ATS*/*ERS Recommendations for Standardized Procedures for the Online and Offline Measurement of Exhaled Lower Respiratory Nitric Oxide and Nasal Nitric Oxide* (Am J Respir Crit Care Med Vol 171, pp 912-930, 2005) empfohlen wird. Gegenüber passiven Systemen, die ohne Pumpen arbeiten, hat die erfindungsgemäße Vorrichtung bei Verwendung von ein oder mehreren Pumpen daher den Vorteil, dass geeignete Druckverhältnisse und Strömungsmengen sehr genau eingestellt werden können.

Da erfindungsgemäß nur ein Nebenstrom oder Teilstrom, beispielsweise 10 % der Ausatemluft, in oder durch die Messkammer bzw. die Gassensoreinrichtung geleitet wird, hat die erfindungsgemäße Vorrichtung insgesamt den Vorteil, dass das System verhältnismäßig klein und damit im Allgemeinen kostengünstig ausgelegt werden kann. Beispielsweise kann das Umschaltventil, das bei der oben beschriebenen Variante mit einer gemeinsamen Pumpe für den Messgaspfad und den Spülgaspfad erforderlich ist, im Vergleich mit anderen Lösungen wesentlich kleiner und damit kostengünstiger ausgelegt werden. Auch andere Komponenten, wie beispielsweise Leitungen, die Pumpe(n), die Messkammer und die darin enthaltenen Komponenten, können im Vergleich mit anderen Systemen kleiner ausgelegt werden.

Zweckmäßigerweise weist die erfindungsgemäße Vorrichtung ein Mundstück auf, über das die Ausatemluft des Anwenders in die Vorrichtung eingeblasen wird. Hierbei kann es sich insbesondere um ein austauschbares Mundstück handeln, das beispielsweise nach einer bestimmten Anzahl von Verwendungen oder nach einer bestimmten Zeit ausgetauscht bzw. erneuert wird. In besonders bevorzugter Weise ist das Mundstück mit einem oder mehreren Entfeuchtern ausgestattet, die beispielsweise Silikagel oder Wasser bindende Salze enthalten. Eine Entfeuchtung der Atemluft ist besonders vorteilhaft, da eine Kondensation von Feuchtigkeit in der Messkammer das Messergebnis verfälschen kann. Im Zusammenhang mit der Erfindung hat eine Entfeuchtung der Ausatemluft im Bereich des Mundstückes weiterhin den besonderen Vorteil, dass auf diese Weise auch die Spülluft, die durch dieses Mundstück in die Vorrichtung eintritt, entfeuchtet ist. Bisherige Vorrichtungen beziehen die Spülluft in der Regel direkt aus der Umgebungsluft, sodass die Spülluft in der Regel nicht entfeuchtet ist oder separat entfeuchtet werden muss. Durch eine ausreichende Entfeuchtung der Spülluft im Mundstück wird vermieden, dass eine Kondensation von Feuchtigkeit aus der Spülluft in der Messkammer die Genauigkeit der Stickstoffoxidmessung negativ beeinflusst.

Weiterhin kann das Mundstück einen oder mehrere Keimfilter enthalten. Dies ist insbesondere im Hinblick auf hygienische Anforderungen an die Vorrichtung vorteilhaft, da hierdurch Kontaminationen und Verkeimungen der Vorrichtung bei längerem Gebrauch vermieden werden.

In einer besonders bevorzugten Ausgestaltung der erfindungsgemäßen Vorrichtung kann der Anwender über das Mundstück Umgebungsluft, vorzugsweise gefilterte Umgebungsluft, einatmen. Hierfür mündet in das Mundstück ein Zuführpfad für Umgebungsluft. Mit besonderem Vorteil ist in diesem Pfad wenigstens ein Filter (Null-Luft-Filter) zur Generierung von im Wesentlichen schadstofffreier Luft vorgesehen, sodass der Anwender schadstofffreie Luft einatmen kann, bevor er die zu messende Ausatemluft in das Gerät einbläst. Hierdurch wird ein Offset der Messergebnisse durch Schadstoffe aus der Umgebungsluft ausgeschlossen. Bei dem Filter kann es sich beispielsweise um einen Aktivkohlefilter handeln, der insbesondere Stickstoffoxide und/oder alkoholische Komponenten und/oder Kohlenstoffmonoxid aus der Umgebungsluft herausfiltert. Optional kann in diesem Zuführpfad für Umgebungsluft ein Rückschlagventil vorgesehen sein, um den Filter bei der Lagerung vor Kontaminationen zu schützen.

Die Abzweigung des Messgaspfades und des Spülgaspfades von dem Hauptgaspfad ermöglicht den Einsatz von verschiedenen Gassensoreinrichtungen, die entweder auf einer Messung in einer kontinuierlich durchflossenen Messkammer beruhen oder die auf einer Messung mit einem abgeschlossenen Probenvolumen beruhen. Für die Gassensoreinrichtung können daher im Prinzip verschiedene Gassensoren, die auf unterschiedlichen Prinzipien basieren und die für den Nachweis und die Messung von unterschiedlichen Komponenten in der Ausatemluft eingerichtet sind, eingesetzt werden.

Mit besonderem Vorteil ist die erfindungsgemäße Vorrichtung für die Messung von Stickstoffoxiden in der Ausatemluft eingerichtet. Hierfür kann ein Stickoxidgassensor eingesetzt werden, der beispielsweise auf absorptionspektroskopischer Basis direkt Stickstoffmonoxid in der Ausatemluft misst. Besonders bevorzugt ist es, eine Gassensoreinrichtung zu verwenden, die mit einer Konvertereinrichtung gekoppelt ist, die zumindest teilweise eine Umwandlung des Stickstoffmonoxids aus der Ausatemluft in Stickstoffdioxid bewirkt. In dieser Ausgestaltung sind zweckmäßigerweise dann ein oder mehrere Gassensoren in der Gassensoreinrichtung vorgesehen, die zur Messung von Stickstoffdioxid geeignet sind. Für die Umwandlung von Stickstoffmonoxid zu Stickstoffdioxid können in an sich bekannter Weise in der Konvertereinrichtung geeignete Oxidationsmittel vorgesehen sein, oder die Umwandlung in Stickstoffdioxid erfolgt mittels eines geeigneten Katalysators.

Der Gassensor kann beispielsweise nach dem Prinzip der Austrittsarbeitsmessung arbeiten, wobei vorzugsweise ein Feldeffekttransistor verwendet wird. Eine geeignete Gassensoreinrichtung geht beispielsweise aus der eingangs bereits genannten internationalen Patentanmeldung WO 2002/088691 A2 hervor. Ein Sensor in dieser Art hat den besonderen Vorteil, dass der Sensor sehr klein und energiesparend ausgestaltet sein kann. Zudem ist der Sensor nicht verschleißend, sodass er in besonderer Weise für die erfindungsgemäße Vorrichtung geeignet ist.

Zweckmäßigerweise weist die erfindungsgemäße Vorrichtung eine geeignete Sensorik für eine Kontrolle des Gasflusses und der Volumenströme in der Vorrichtung auf. Es können insbesondere ein oder mehrere Sensoren zur Messung der Luftmenge und/oder des Drucks und/oder des Gasflusses vorgesehen sein. Auf diese Weise kann eine optimale Luftfraktion oder Luftmenge der in das Gerät eingeblasenen Ausatemluft für die Messung und für das Spülen verwendet werden. Eine geeignete Sensorik erlaubt damit eine optimale Steuerung der Probenentnahme für kontrollierte Messungen. Die Sensoren können übliche Durchflusssensoren und/oder Drucksensoren sein. Beispielsweise kann eine Differenzdruckmessung an einer Engstelle im Hauptgaspfad durchgeführt werden, wobei beispielsweise stromaufwärts und stromabwärts einer Engstelle jeweils ein Drucksensor angeordnet ist.

Darüber hinaus können weitere Sensoren vorhanden sein, insbesondere ein oder mehrere Sensor(en) zur Feuchtigkeitsmessung. Eine ausreichende Entfeuchtung der Luft kann für die Zuverlässigkeit der Messergebnisse wichtig sein. Durch einen Feuchtigkeitssensor kann eine ausreichende Entfeuchtung der Ausatemluft überprüft und kontrolliert werden. Weiterhin können gemessene Werte zur Feuchtegehalt des Messgases bei der Auswertung der Messdaten berücksichtigt werden. Ein Feuchtigkeitssensor kann beispielsweise stromaufwärts der Abzweigung des Spülluftpfades angeordnet sein, sodass damit sichergestellt werden kann, dass auch die Spülluft ausreichend entfeuchtet ist. Durch eine ausreichende Entfeuchtung der Spülluft wird vermieden, dass eine Kondensation von Feuchtigkeit aus der Spülluft in der Messkammer die Genauigkeit der Stickstoffoxidmessung negativ beeinflusst.

Schließlich umfasst die Erfindung die Verwendung der beschriebenen erfindungsgemäßen Vorrichtung zur Messung von Stickstoffoxiden in der Ausatemluft. Da die Stickstoffoxide und insbesondere Stickstoffmonoxid in der Ausatemluft einer Person oder gegebenenfalls eines Tieres ein wichtiger Indikator für den Verlauf von asthmatischen Erkrankungen und allgemein für entzündliche Reaktionen in den Atemwegen oder in der Lunge sind, kann mit der erfindungsgemäßen Vorrichtung der Verlauf einer asthmatischen Erkrankung gemessen werden. Insbesondere kann die aktuelle Reaktion des Körpers überwacht werden, um gegebenenfalls entsprechend, beispielsweise durch eine Medikamentengabe, reagieren zu können. Bezüglich weiterer Merkmale der Vorrichtung, die zu diesem Zweck verwendet wird, wird auf die obige Beschreibung verwiesen.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

In den Zeichnungen zeigen:
- Fig. 1: eine bevorzugte Ausgestaltung einer erfindungsgemäßen Vorrichtung zur Messung von Stickstoffoxiden in der Ausatemluft und
- Fig. 2: eine weitere bevorzugte Ausgestaltung einer erfindungsgemäßen Vorrichtung zur Messung von Stickstoffoxiden in der Ausatemluft.

### Beschreibung von Ausführungsbeispielen

Bei der erfindungsgemäßen Vorrichtung handelt es sich um ein Atemgasanalysegerät, bei dem eine Person oder gegebenenfalls ein Tier, insbesondere ein Patient, in das Gerät bläst. Ein Teil der Ausatemluft wird in eine Messkammer mit einem Sensor geleitet, wobei in der Messkammer direkt oder indirekt der Stickstoffmonoxidgehalt oder gegebenenfalls andere Komponenten in der Ausatemluft bestimmt werden. Ein Teil der Luft, der nicht in die Messkammer geleitet wird, wird über einen Hauptgaspfad wieder aus dem Gerät geleitet, sodass ein angenehmer Ausatemvorgang für den Patienten möglich ist. Erfindungswesentlich ist hierbei, dass von dem Hauptgaspfad zum einen der Messgaspfad, der eine Fraktion der Ausatemluft in die Messkammer leitet, abzweigt. Weiterhin zweigt von dem Hauptgaspfad auch ein Spülgaspfad ab, der insbesondere gefilterte Luft in die Messkammer führt, um dort eine Spülung und gegebenenfalls einen Nulllinienabgleich oder eine Kalibrierung des Sensors vornehmen zu können. Die Probenentnahme für das Messgas und für die Spülluft erfolgt über wenigstens eine Pumpe. Gegebenenfalls können zwei Pumpen vorgesehen sein, jeweils eine Pumpe im Messgaspfad und eine Pumpe im Spülgaspfad. Durch das aktive Pumpen der jeweiligen Gasfraktionen ist eine genaue Steuerung der Messung möglich, wobei eine genau abgestimmte Luftfraktion und ein definiertes Luftvolumen verwendet werden können, die für die Messung optimal sind. Insgesamt wird dadurch die Stickstoffoxidmessung vereinfacht und präzisiert, da eine genaue Steuerung und Kontrolle der Probenentnahme im Hinblick auf die Zeit und die Menge über die Pumpe(n) möglich ist. Zweckmäßigerweise wird nur ein kleiner Anteil, beispielsweise 10 %, der Atemluft, in den Messgaspfad geleitet. Dadurch kann der Messgaspfad inklusive seiner Komponenten (Leitungen, Ventile, Pumpen, Messkammer, gegebenenfalls Konvertereinrichtung etc.) im Vergleich mit herkömmlichen Einrichtungen wesentlich kleiner ausgelegt werden. Die erfindungsgemäße Vorrichtung bzw. das Atemgasanalysegerät kann damit kostengünstiger hergestellt werden und es kann für einen handlichen Gebrauch ausgelegt werden. Wenn eine Konvertereinrichtung für die Umwandlung von Stickstoffmonoxid vorgesehen ist, kann die Konvertereinrichtung wesentlich kleiner ausgelegt werden und es ist wesentlich weniger Oxidationsmittel oder Katalysatormaterial erforderlich im Vergleich mit sonst üblichen passiven Lösungen.

Ein besonderer Vorteil der erfindungsgemäßen Lösung ist, dass eine Kondensation von Feuchtigkeit in der Messkammer, die zu einer Verfälschung des Messergebnisses durch eine Absorption von Stickstoffdioxid in dem kondensierten Wasser auftreten kann, vermieden wird. Dies wird dadurch erreicht, dass die Spülluft aus dem Hauptgaspfad stammt, wo eine Entfeuchtung stattfindet oder bereits stattgefunden hat. Die Entfeuchtung kann vorzugsweise bereits im Mundstück erfolgen. Bei bisherigen Lösungen aus dem Stand der Technik wird in der Regel zwar die Atemluft, die für die Messung verwendet wird, entfeuchtet. Die Spülluft, die aus der Umgebung stammt, wird jedoch nicht entfeuchtet.

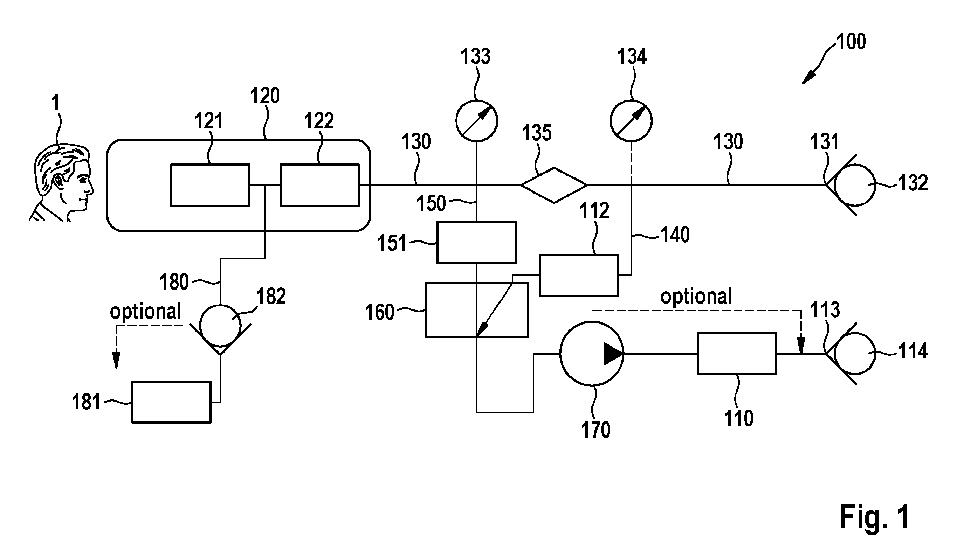

**Fig. 1** illustriert in schematischer Weise eine Ausgestaltungsmöglichkeit des erfindungsgemäßen Atemgasanalysegeräts 100. Die Vorrichtung 100 umfasst eine Messkammer 110 als Gassensoreinrichtung, die einen Gassensor zur Messung von Stickstoffdioxid enthält. Weiterhin ist der Messkammer 110 eine Konvertereinrichtung 112 zugeordnet, wobei die Konvertereinrichtung 112 eine Umwandlung des Stickstoffmonoxids aus der Ausatemluft in Stickstoffdioxid bewirkt. Die Konvertereinrichtung kann sich an unterschiedlichen Positionen in der Vorrichtung und insbesondere an unterschiedlichen Positionen im Messgaspfad 140 befinden. Die Vorrichtung 100 umfasst weiterhin ein Mundstück 120, insbesondere ein austauschbares Mundstück oder ein Einwegmundstück. Das Mundstück 120 beinhaltet vorzugsweise einen Keimfilter 121 und eine Entfeuchtungseinheit 122.

Eine Person bzw. ein Patient 1 bläst in das Mundstück 120 die Ausatemluft ein. Der Luftstrom wird über den Hauptgaspfad 130 durch das Gerät bis zu einer Austrittsöffnung 131 geleitet, die mit einem Rückschlagventil 132 gegen Kontaminationen bei der Lagerung gesichert ist. Das Rückschlagventil 132 verhindert darüber hinaus auch beim Einatmen durch den weiter unten noch zu erläuternden Einatemfilter 181 unerwünschten ungefilterten Lufteintritt ins Gerät. Von dem Hauptgaspfad 130 zweigen ein Spülgaspfad 150 und ein Messgaspfad 140 ab. Zwischen dem Messgaspfad 140 und dem Spülgaspfad 150 kann mit einem Umschaltventil 160 umgeschaltet werden. Über den Messgaspfad 140 bzw. den Spülgaspfad 150 wird eine Fraktion der Ausatemluft von dem Hauptgaspfad 130 abgezweigt und in die Messkammer 110 geleitet. Für die Messung oder die Spülung kann beispielsweise ein Anteil von 10 % der Ausatemluft abgezweigt und durch die Messkammer 110 geleitet werden. Im Spülgaspfad 150 ist ein Filter 151 (Null-Luft-Filter) zur Generierung von im Wesentlichen schadstofffreier Luft, beispielsweise ein Aktivkohlefilter, vorgesehen. Mit der gefilterten Luft erfolgen die Spülung und gegebenenfalls ein Nulllinienabgleich des Gassensors in der Messkammer 110.

Nach dem Durchlaufen der Messkammer 110 verlässt die Messluft oder gegebenenfalls die Spülluft das Gerät durch die Austrittsöffnung 113, die durch ein Rückschlagventil 114 gegen Kontaminationen bei der Lagerung gesichert ist. Der Gasfluss über den Messgaspfad 140 bzw. über den Spülluftpfad 150 wird durch die Pumpe 170 gesteuert. In dieser Ausgestaltung ist die Pumpe stromaufwärts der Messkammer 110 angeordnet. Es kann mit Vorteil jedoch auch vorgesehen sein, dass die Pumpe 170 stromabwärts der Messkammer 110 angeordnet ist. Hierbei wird zum einen die Verschmutzung der Messkammer 110 durch Kontaminationen aus der Pumpe 170 vermieden. Darüber hinaus kann durch diese Anordnung ein Unterdruck in der Messkammer 110 erzeugt werden, der die Spülung und die Regenerierung des Sensors in der Messkammer beschleunigen kann.

Zur optimalen Kontrolle und Steuerung des Systems sind weiterhin Sensoren 133 und optional 134 im Hauptgaspfad 130 vorgesehen, die stromaufwärts und stromabwärts einer Drossel 135 angeordnet sind. Der optionale Sensor 134 ist durch eine gestrichelte Linie angedeutet. Bei den Sensoren 133 und 134 handelt es sich insbesondere um Drucksensoren, über die beispielsweise eine Differenzdruckmessung durchgeführt werden kann, sodass der Gasfluss oder Volumenstrom kontrolliert werden kann. Die Luftmenge wird dabei durch den Druckabfall an der Engstelle 135 im Hauptgaspfad 130 gemessen. Es ist auch möglich, dass nur in der Position 133 oder an anderer Stelle ein oder mehrere Sensor(en) vorgesehen sind.

Optional kann in das Mundstück ein weiterer Pfad 180 (Einatempfad) zur Zufuhr von Umgebungsluft münden. Über den Pfad 180 bzw. diesen Einlass kann die Person 1 Umgebungsluft einatmen, die mittels eines Filters 181 (Null-Luft-Filter) als Einatemfilter gereinigt und von Schadstoffen befreit wird. Ein Rückschlagventil 182 schützt den Filter 181 bei der Lagerung vor Kontaminationen. Das Rückschlagventil 182 kann stromaufwärts oder stromabwärts des Filters 181 angeordnet sein. Durch das dadurch mögliche Einatmen von schadstofffreier Luft kann ein Offset der Messergebnisse durch Schadstoffe aus der Umgebungsluft ausgeschlossen werden.

Die Vorrichtung bzw. das Atemgasanalysegerät kann beispielsweise so ausgelegt sein, dass bei einer Inhalation ein Druckabfall von 3 bis 5 mbar und bei der Ausatmung ein Druckabfall zwischen 5 bis 20 mbar stattfinden.

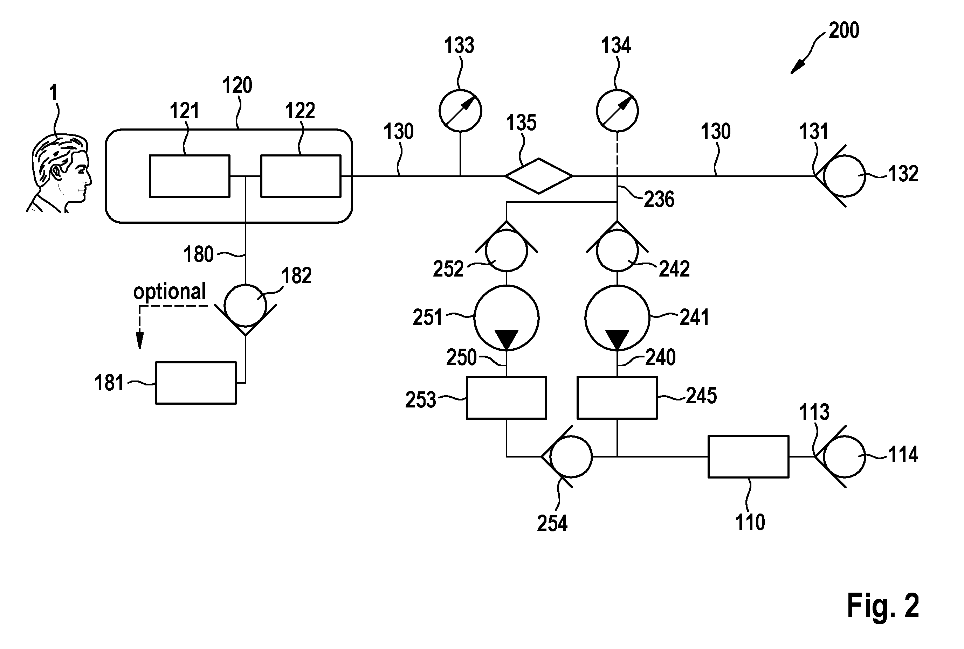

**Fig. 2** illustriert ein weiteres Beispiel für eine Ausgestaltung des erfindungsgemäßen Atemgasanalysegeräts 200, bei dem sowohl im Messgaspfad 240 als auch im Spülgaspfad 250 jeweils eine Pumpe 241, 251 vorgesehen ist. Mit Ausnahme der Ausgestaltung des Messgaspfades 240 und des Spülgaspfades 250 entspricht die Vorrichtung 200 der in der Fig. 1 illustrierten Vorrichtung 100. Die entsprechenden Elemente sind daher mit den gleichen Bezugszeichen wie bei der Vorrichtung 100 bezeichnet und es wird diesbezüglich auf die obige Beschreibung Bezug genommen. Anders als bei der Vorrichtung 100 zweigt bei der Vorrichtung 200 nur stromabwärts der Drossel 135 ein Pfad 236 ab, der sich in den Spülgaspfad 250 und den Messgaspfad 240 verzweigt. Sowohl im Messgaspfad 240 als auch im Spülgaspfad 250 ist jeweils eine Pumpe 241 bzw. 251 vorgesehen, der jeweils ein Rückschlagventil 242 bzw. 252 vorgeschaltet ist. Stromabwärts der Pumpe 251 im Spülgaspfad 250 befindet sich ein Filter 253 zur Generierung von im Wesentlichen schadstofffreier Luft, insbesondere ein Aktivkohlefilter. Stromabwärts dieses Filters befindet sich ein weiteres Rückschlagventil 254. Stromabwärts der Pumpe 241 im Messgaspfad 240 befindet sich die Konvertereinrichtung 245. Je nach Ansteuerung der Pumpen 251 bzw. 241 wird eine Fraktion der Ausatemluft von dem Hauptgaspfad 130 über den Spülgaspfad 250 bzw. den Messgaspfad 240 abgeleitet. Das Spülgas bzw. das Messgas gelangt dann in die Messkammer 110 zur Messung bzw. zur Spülung, bevor es über den Auslass 113 das Gerät verlässt.

In anderen Ausgestaltungen der erfindungsgemäßen Vorrichtung ist es auch möglich, dass in einer Ausführungsform mit zwei Pumpen, also einer Pumpe für den Spülgaspfad und einer Pumpe für den Messgaspfad, einer dieser Pfade stromaufwärts einer Drossel im Hauptgaspfad und der andere Pfad stromabwärts der Drossel abzweigen. Auch die Reihenfolge der abzweigenden Pfade ist im Prinzip frei wählbar. Dies gilt auch für Ausführungsformen mit nur einer Pumpe und einem Umschaltventil. Auch die Positionierung des Filters im Spülgaspfad und der verschiedenen Rückschlagventile kann unterschiedlich erfolgen und beispielsweise in Anpassung an die Platzverhältnisse im Gerätedesign gewählt werden.

## Patentansprüche

1. Vorrichtung (100; 200) zur Analyse der Ausatemluft mit einer Gassensoreinrichtung (110), **dadurch gekennzeichnet, dass** zur Führung von Luft in der Vorrichtung ein Hauptgaspfad (130) vorgesehen ist, von dem wenigstens ein Messgaspfad (140; 240) und wenigstens ein Spülgaspfad (150; 250) abzweigen.

2. Vorrichtung nach Anspruch 1, **dadurch gekennzeichnet, dass** in dem Spülgaspfad (150; 250) wenigstens ein Filter (151; 253) zur Generierung von im Wesentlichen schadstofffreier Luft, insbesondere ein Aktivkohlefilter, vorgesehen ist.

3. Vorrichtung nach Anspruch 1 oder Anspruch 2, **dadurch gekennzeichnet, dass** zur Entnahme von Gas aus dem Hauptgaspfad (130) in den Messgaspfad (140; 240) und in den Spülgaspfad (150; 250) wenigstens eine Pumpe (170; 241, 251) vorgesehen ist.

4. Vorrichtung nach einem der vorhergehenden Ansprüche, **dadurch gekennzeichnet, dass** zur Umschaltung zwischen Messgaspfad (140) und Spülgaspfad (150) ein Umschaltventil (160) vorgesehen ist.

5. Vorrichtung nach Anspruch 3 oder Anspruch 4, **dadurch gekennzeichnet, dass** die Pumpe stromabwärts der Gassensoreinrichtung (110) angeordnet ist.

6. Vorrichtung nach einem der Ansprüche 1 bis 3, **dadurch gekennzeichnet, dass** in dem Messgaspfad (240) und in dem Spülgaspfad (250) jeweils wenigstens eine Pumpe (241, 251) vorgesehen ist.

7. Vorrichtung nach einem der Ansprüche 3 bis 6, **dadurch gekennzeichnet, dass** die Pumpe oder die Pumpen (170; 241, 251) Membranpumpen und/oder piezokeramische *Microblower* sind.

8. Vorrichtung nach einem der Ansprüche 3 bis 7, **dadurch gekennzeichnet, dass** der Pumpe oder den Pumpen (241, 251) jeweils wenigstens ein Rückschlagventil (242, 252) zugeordnet ist.

9. Vorrichtung nach einem der vorhergehenden Ansprüche, **dadurch gekennzeichnet, dass** die Vorrichtung ein Mundstück (120), insbesondere ein austauschbares Mundstück, zum Einbringen der Ausatemluft in die Vorrichtung umfasst, wobei vorzugsweise das Mundstück wenigstens einen Entfeuchter (122) und/oder wenigstens einen Keimfilter (121) umfasst.

10. Vorrichtung nach Anspruch 9, **dadurch gekennzeichnet, dass** in das Mundstück (120) ein Pfad (180) zur Zufuhr von Umgebungsluft mündet, wobei vorzugsweise in dem Pfad (180) wenigstens ein Filter (181) zur Generierung von im Wesentlichen schadstofffreier Luft, insbesondere ein Aktivkohlefilter, vorgesehen ist.

11. Vorrichtung nach Anspruch 10, **dadurch gekennzeichnet, dass** dem Filter (181) wenigstens ein Rückschlagventil (182) zugeordnet ist.

12. Vorrichtung nach einem der vorhergehenden Ansprüche, **dadurch gekennzeichnet, dass** die Vorrichtung zur Messung von Stickstoffoxiden in der Ausatemluft vorgesehen ist und dass die Gassensoreinrichtung (110) wenigstens einen Gassensor zur Messung von Stickstoffoxiden umfasst, wobei vorzugsweise der Gassensoreinrichtung eine Konvertereinrichtung (112; 245) zur zumindest teilweisen Umwandlung von Stickstoffmonoxid in Stickstoffdioxid zugeordnet ist.

13. Vorrichtung nach einem der vorhergehenden Ansprüche, **dadurch gekennzeichnet, dass** die Gassensoreinrichtung (110) einen Feldeffekttransistor umfasst.

14. Vorrichtung nach einem der vorhergehenden Ansprüche, **dadurch gekennzeichnet, dass** die Vorrichtung einen oder mehrere Sensoren (133, 134) zur Messung der Luftmenge und/oder des Drucks und/oder des Gasflusses und/oder der Feuchtigkeit umfasst.

15. Verwendung einer Vorrichtung (100; 200) gemäß einem der Ansprüche 1 bis 14 zur Messung von Stickstoffoxiden in der Ausatemluft.
